# EUROPÄISCHE PATENTSCHRIFT

(11) **EP 1 247 800 B1**
(45) Veröffentlichungstag und Bekanntmachung des Hinweises auf die Patenterteilung: **22.03.2006**
(21) Anmeldenummer: 02010124.2
(22) Anmeldetag: 23.03.1998
(51) Int. Cl.: C07C 229/24, C07C 227/08, D21C 9/10

(54) **Verwendung von Iminodibernsteinsäuresalzen bei der Papierherstellung**
Use of iminodisuccinic acid salts in the production of paper
Utilisation de sels d'acide iminodisuccinique dans la production de papier

(30) Priorität: 04.04.1997 DE 19713911
(43) Veröffentlichungstag der Anmeldung: 09.10.2002
(62) Teilanmeldung aus: 98917048.5
(73) Patentinhaber: LANXESS Deutschland GmbH, 51369 Leverkusen (DE)
(72) Erfinder: Groth, Torsten, Dr., 51519 Odenthal (DE); Joentgen, Winfried, Dr., 50735 Köln (DE); Wagner, Paul, Dr., 40597 Düsseldorf (DE); Döbert, Frank, Dr., 50933 Köln (DE); Wenderoth, Eckard, 51381 Leverkusen (DE); Roick, Thomas, Dr., 51375 Leverkusen (DE)

(56) Entgegenhaltungen:
- EP-A- 0 509 382
- EP-A- 0 831 165
- DE-A- 4 340 043
- DATABASE WPI Section Ch, Week 199612 Derwent Publications Ltd., London, GB; Class D25, AN 1996-112673 XP002069427 & JP 08 012631 A (NITTO CHEM IND CO LTD), 16. Januar 1996 (1996-01-16)

## Beschreibung

Die Erfindung betrifft die Verwendung von Iminodibernsteinsäurealkalisalzen erhältlich durch Umsetzung von Maleinsäure und Ammoniak in wässrigem Medium in Gegenwart von Alkalimetallhydroxiden bei der Papierherstellung.

Komplexiermittel werden seit Jahren in großen Mengen eingesetzt. Viele bisher gebräuchliche Komplexiermittel, wie Ethylendiamintetraessigsäure (EDTA), Diethylentriaminpentaessigsäure (DTPA), Nitrilotriessigsäure (NTA) und verschiedene Phosphonate sind nicht oder nur bedingt biologisch abbaubar, remobilisieren Schwermetalle in Oberflächengewässem und können sogar bis in die Trinkwasseraufbereitung gelangen, da sie weder in Klärschlämmen noch in Böden adsorbiert werden. Phosphate sind Komplexiermittel, die zur Eutrophierung von Gewässern beitragen. Zusammengefasst handelt es sich dabei um ökotoxikologische Eigenschaften, die in der heutigen Zeit als nachteilig empfunden werden.

Daher ist es eine wichtige Aufgabe, Komplexiermittel zu entwickeln, die die bisherigen ökotoxikologischen Nachteile nicht aufweisen. Iminodibernsteinsäure ist nun ein Komplexiermittel, das eine leichte biologische Abbaubarkeit zeigt und damit gegenüber den bisherigen Komplexiermitteln einen ökotoxikologischen Vorteil besitzt.

Zukünftig werden aber nicht nur die Produkteigenschaften von Chemikalien, die nach dem Gebrauch überwiegend in die Umwelt gelangen, unter den geschilderten Aspekten kritisch geprüft, sondern auch die Herstellungsverfahren. So war es überraschend, dass für eine industriell zur Zeit noch nicht verfügbare Chemikalie, die umweltgerechte Eigenschaften besitzt, auch ein umweltgerechtes Herstellungverfahren gefunden werden konnte.

Für Iminodibernsteinsäure sind auf der Basis von Maleinsäureanhydrid oder Maleinsäure und Ammoniak bisher folgende Herstellungsmöglichkeiten bekannt: In GB 1 306 331 wird die Herstellung von Iminodibernsteinsäure aus Maleinsäure und Ammoniak im Molverhältnis von 2:3 bis 2:5 bei Temperaturen von 60 bis 155°C beschrieben. Zur Aufarbeitung werden entweder Salzsäure oder Natronlauge hinzugegeben. In SU 0 639 863 wird Iminodibernsteinsäure in Gegenwart von Alkalimetallhydroxiden aus Maleinsäure und Ammoniak bei einem Molverhältnis von 2:0,8 bis 2:1 und Temperaturen von 110 bis 130°C hergestellt. In JP 6/329 606 wird ein dreistufiges Verfahren zur Herstellung von Iminodibernsteinsäure beschrieben. Zunächst reagiert ein Maleinsäurederivat mit Ammoniak in wässrigem Medium. Danach erfolgt die Zugabe von Alkali- oder Erdalkalimetallhydroxiden. In der dritten Verfahrensstufe schließt sich ein sogenannter Reifungsprozess an. In JP 6/329 607 wird ebenfalls ein dreistufiges Verfahren zur Herstellung von Iminodibernsteinsäure beschrieben. In der ersten Stufe wird wiederum zunächst ein Maleinsäurederivat mit Ammoniak in wässrigem Medium umgesetzt. Danach erfolgt in der zweiten Stufe der Zusatz von Alkali- oder Erdalkalimetallhydroxiden. In der dritten Stufe wird nach Zusatz von weiterem Maleinsäurederivat die Reaktion fortgesetzt. In dieser Patentanmeldung wird ausdrücklich gesagt, dass als Maleinsäurederivate Maleinsäureanhydrid, Maleinsäure oder das Maleinsäureammoniumsalz eingesetzt werden. Mit den Metallsalzen der Maleinsäure soll die gewünschte Reaktion kaum stattfinden, so dass man das Ziel nicht erreichen kann.

EP-A 0 831 165 offenbart Iminodibernsteinsäuren zur Vorbehandlung von Fasern als Bleichregulatoren in der H₂O₂-Bleiche von textilbildenden natürlichen pflanzlichen Fasern.

Auch DE 4 340 043 A beschreibt die Verwendung von Stickstoff-haltigen Komplexbildnern bei der Holzstoffbleiche mit H₂O₂.

Die Erfindung betrifft bevorzugt die Verwendung von Iminodibernsteinsäurealkalisalzen bei der Papierherstellung erhältlich durch Umsetzung von Maleinsäureanhydrid (MSA), Alkalimetallhydroxid (MeOH), Ammoniak (NH₃) und Wasser im Molverhältnis von MSA : MeOH : NH₃ : H₂O = 2:0,1-4:1,1-6:5-30 bei Temperaturen von 70-170°C, unter Drücken von 1-80 bar und Reaktionszeiten von 0,1-100 h, Abdestillieren von Ammoniak und Wasser aus dem Umsetzungsgemisch unter Zusatz von Wasser und 0-4 Mol MeOH pro 2 Mol ursprünglich eingesetztem MSA bei Temperaturen von 50-170°C unter Drücken von 0,1-50 bar innerhalb von 0,1-50 h und Zusetzen von Wasser nach der Destillation in einer solchen Menge, dass die entstehende Lösung einen Feststoffgehalt von 5-60 %, bezogen auf das Gesamtgewicht der Lösung, enthält.

Im Herstellverfahren werden in einem Reaktor Wasser, Alkalimetallhydroxid (MeOH), Maleinsäureanhydrid (MSA) und Ammoniak (NH₃) eindosiert und das entstandene Maleinsäuresalz bei den genannten Reaktionstemperaturen (T) und Reaktionszeiten (t) umgesetzt. Anschließend werden unter Zusatz von Wasser und gegebenenfalls weiterem MeOH Ammoniak im Gemisch mit Wasser abdestilliert. Nach dieser Destillation wird das Produkt durch Zugabe von Wasser auf eine zweckmäßige Konzentration eingestellt. Diese Produktlösung kann gegebenenfalls einer Klärfiltration unterzogen werden. Me bedeutet hierbei Li, Na oder K, bevorzugt Na oder K, besonders bevorzugt Na.

Das Herstellverfahren hat den Vorteil, dass es sowohl diskontinuierlich als auch kontinuierlich durchgeführt und dabei ein hohes Maß an Wirtschaftlichkeit erreicht werden kann. Dieser Sachverhalt ist von großer Bedeutung, da auch umweltfreundliche Produkte trotz aller Vorteile nur konkurrenzfähig sind, wenn sie unter entsprechend ökonomischen Bedingungen hergestellt werden können. Das erfindungsgemäße Verfahren erzeugt keinen Abfall, da nach der Destillation des Ammoniaks, der recycliert und weiter verwertet und gegebenenfalls wieder eingesetzt werden kann, das verbleibende Produkt vollständig verwendet wird. Dieses Produkt ist darüber hinaus nach OECD 301 E leicht biologisch abbaubar. Im Verfahren und Produkt werden Ökonomie und Ökologie in bisher nicht bekannter Weise miteinander vereint.

Im Herstellverfahren werden zunächst MSA, Wasser und Alkalimetallhydroxid im Molverhältnis von MSA : MeOH : Wasser = 2:0,1-4:5-30 miteinander vermischt, wobei unterschiedliche Dosiervarianten ausgeführt werden können. So kann MSA zunächst mit Wasser über die Stufe der Maleinsäure oder alternativ direkt mit wässriger Alkalimetallhydroxidlösung zu den entsprechenden Maleinsäuresalzen umgewandelt werden. Aus technischen und chemischen Gründen hat sich die zweite Dosiervariante als vorteilhaft erwiesen. Mit ihr ist es möglich, auf einfache Weise besonders konzentrierte und an Nebenkomponenten arme Maleinsäuresalzlösungen herzustellen. Diese leicht gelblichen Lösungen enthalten die möglichen Nebenkomponenten Fumarsäure und Äpfelsäure nur in geringen Mengen. So wird das Maleinsäuresalz mit Ausbeuten von >92 %, bevorzugt >95 %, besonders bevorzugt >98 % der theoretischen Menge im weiteren Prozess verwendet.

Im Hinblick auf eine kontinuierliche Prozessführung hat sich die kontinuierliche und simultane Dosierung von MSA und Alkalimetallhydroxidlösungen in eine vorgelegte, Maleinsäuresalzlösung als besonders vorteilhaft erwiesen. Auf diese Weise können sogar sehr reine und auch farblose Lösungen mit ebenso hohen Ausbeuten erhalten werden.

In bevorzugter Weise werden MSA und MeOH in einem Molverhältnis von 2:0,5-3,9, besonders bevorzugt 2:0,9-3,5, ganz besonders bevorzugt 2:1,5-3,1, eingesetzt.

In bevorzugter Weise werden MSA und NH3 in einem Molverhältnis von 2:1,2-5,5, besonders bevorzugt 2:1,5-4,5, ganz besonders bevorzugt 2:1,9-3,5, eingesetzt.

In bevorzugter Weise werden MSA und H₂O in einem Molverhältnis von 2:5,5-25, besonders bevorzugt 2:6-20, ganz besonders bevorzugt 2:6,5-15, eingesetzt.

Die Herstellung des Maleinsäuresalzes aus MSA, MeOH und Wasser erfolgt bei Temperaturen von mindestens 60°C, beispielsweise bei 60-130°C, bevorzugt bei 70-120°C, besonders bevorzugt bei 80-115°C, bei denen eine schnelle und vollständige Reaktion des MSA gewährleistet ist und bei denen eine Rührfähigkeit und Pumpfähigkeit der Mischung aufrecht erhalten werden kann. So kann das Maleinsäuresalz als Suspension oder Lösung, bevorzugt als Lösung vorliegen, die außerdem über mehrere Stunden gerührt werden kann, ohne dass nennenswerte Ausbeuteverluste auftreten würden.

Zu den aus MSA, MeOH und Wasser gebildeten Maleinsäuresalz-Suspensionen oder -Lösungen wird Ammoniak in einem Molverhältnis von MSA : Ammoniak = 2:1,1-6, bevorzugt 2:1,2-5,5, besonders bevorzugt 2:1,5-4,5 dosiert. Die Zugabe kann gleichermaßen sowohl in diskontinuierlicher als auch in kontinuierlicher Prozessführung erfolgen.

Die aus MSA, MeOH, Ammoniak und Wasser gebildeten Maleinsäuresalzlösungen werden bei Temperaturen von 70-170°C, bevorzugt bei 80-160°C, besonders bevorzugt bei 85-150°C, ganz besonders bevorzugt 90-145°C und Reaktionszeiten von 0,1-100 h, bevorzugt 0,2-50 h, besonders bevorzugt 0,3-25 h, ganz besonders bevorzugt 0,5-20 h umgesetzt. Die Reaktion kann sowohl in Diskonti- als auch Konti-Reaktoren durchgeführt werden.

Die Reaktion wird in der Regel unter dem sich automatisch einstellenden Druck durchgeführt. Dabei können Drücke von bis zu 50 bar, bevorzugt bis zu 30 bar, besonders bevorzugt bis zu 20 bar auftreten. Eine Überlagerung mit Inertgasen, besonders in Diskonti-Reaktoren, kann dabei zusätzlich vorgenommen werden, wobei Drücke bis zu 80 bar zulässig sind.

Durch die Reaktionsbedingungen wird ein Maleinsäure-Umsatz von >93 %, bevorzugt >95 %, besonders bevorzugt >98 % des theoretischen Umsatzes erreicht.

Nach der Reaktion werden aus dem Umsetzungsgemisch unter Zusatz von Wasser und 0-4 Mol, bevorzugt 0,5-3,5 Mol, besonders bevorzugt 0,7-3,0 Mol, ganz besonders bevorzugt 0,9-2,5 Mol MeOH pro 2 Mol ursprünglich eingesetztem MSA Ammoniak und Wasser abdestilliert. Der Zusatz von Wasser und MeOH kann vor oder während der Destillation sowohl im Diskonti- als auch Konti-Prozess erfolgen. Die Menge des zugesetzten Wassers wird unter Berücksichtigung des unvermeidlich mit dem NH₃ abdestillierenden Wassers so bemessen, dass im verbleibenden Aufarbeitungsgemisch ein Feststoffgehalt von 75 Gew.%, bevorzugt von 70 Gew.-%, besonders bevorzugt von 65 Gew.-%, bezogen auf das Gesamtgewicht des Ansatzes, nicht überschritten wird.

Die Destillation erfolgt bei Temperaturen von 50-170°C, bevorzugt bei 60-150°C, besonders bevorzugt bei 70-140°C, ganz besonders bevorzugt 80-135°C und Drücken von 0,1-50 bar, bevorzugt 0,5-20 bar innerhalb von 0,1-50 h, bevorzugt 0,3-30 h, besonders bevorzugt 0,5-25 h, ganz besonders bevorzugt 0,9-20 h. Dadurch wird eine weitestgehende Abtrennung von nicht umgesetztem und hydrolysierbarem Ammoniak (z.B. durch Kondensation gebildeter Amidstickstoff RCONH2 + MeOH----> RCOOMe + NH3 mit R = Kohlenwasserstoffrest der zugrundeliegenden Carbonsäure) aus dem Umsetzungsgemisch erreicht, der anschließend aufbereitet werden und für eine Weiterverwertung genutzt werden kann. Dadurch wird außerdem eine Minimierung des Stickstoffgehaltes im Produkt erreicht und so bei der Verwendung ein unnötig hoher Stickstoffeintrag in Gewässer vermieden. Der Gehalt der nach der Reaktion noch verbliebenen freien Maleinsäure wird ebenfalls weiter verringert.

Nach der Destillation wird Wasser in einer solchen Menge zugesetzt, dass die entstehende Produktlösung einen Feststoffgehalt, gerechnet als Summe aller Alkalisalze, von 5-60 Gew.-%, bevorzugt 10-58 Gew-.%, besonders bevorzugt 15-55 Gew.-% aufweist. Im Anschluss daran kann im Bedarfsfall eine Klärfiltration durchgeführt werden. Derartige Lösungen sind nahezu geruchsneutral und lagerstabil.

Nach Reaktion und Aufarbeitung werden Iminodibernsteinsäure und ihre Salze (Formel 1) in Ausbeuten von >65 %, bevorzugt >70 %, besonders bevorzugt >74% der theoretischen Ausbeute erhalten. Die Summe aller Nebenkomponenten und ihrer Salze liegen in Mengen von <35 %, bevorzugt <30 %, besonders bevorzugt <26 % der theoretischen Mengen vor, wobei Maleinsäure und ihre Salze (Formel 2) mit <7 %, bevorzugt <5 %, besonders bevorzugt <2 % der theoretischen Menge, Fumarsäure und ihre Salze (Formel 3) mit <20 %, bevorzugt <15 %, besonders bevorzugt <10 % der theoretischen Menge, Äpfelsäure und ihre Salze (Formel 4) mit <7 %, bevorzugt <5 %, besonders bevorzugt <3 % der theoretischen Menge und Asparaginsäure und ihre Salze (Formel 5) mit <25 %, bevorzugt <20 %, besonders bevorzugt <15 % der theoretischen Menge vorliegen. 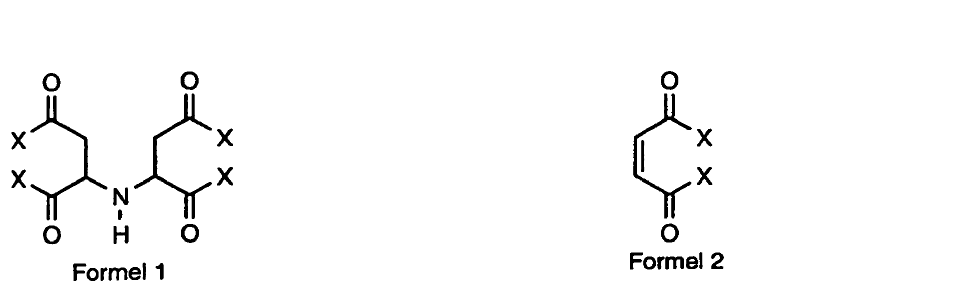 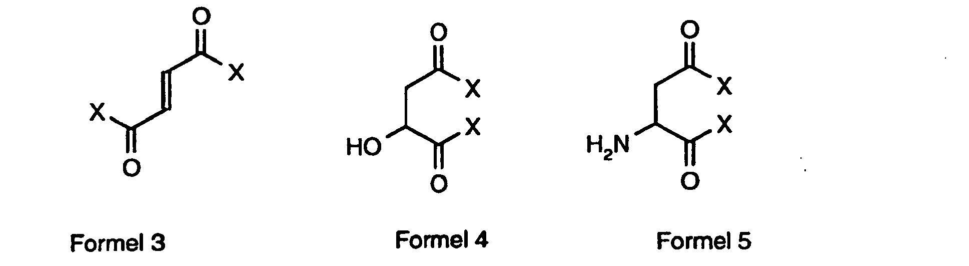 X = OH, OLi, ONa, OK, ONH₄

Insgesamt werden Produktlösungen erhalten, in denen die angegebenen Komponenten der Formeln 1-5 in Summenausbeuten von >93 %, bevorzugt >96 %, besonders bevorzugt >98 % der theoretischen Ausbeute vorliegen. Der Bioabbau der Produkte liegt entsprechend dem OECD 301 E-Test nach 28d über 70 %, meist über 72 %, vielfach über 74 %.

Die Carboxylgruppen von Iminodibernsteinsäure und ihren Nebenkomponenten liegen entsprechend der bei Reaktion und Aufarbeitung eingesetzten MeOH-Menge und der bei der Aufarbeitung abdestillierten Ammoniakmenge in Säure- oder Salzform vor. So kann Iminodibernsteinsäure für den Fall von Me=Na als Na₂- bis Na₄₋Salz, bevorzugt als Na₃- bis Na₄-Salz, besonders bevorzugt als Na₄-Salz erhalten werden, wobei gegebenenfalls die übrigen Carboxylgruppen als freie Säure, sowie Ammoniumsalz vorliegen. Für den Fall des Einsatzes von LiOH beziehungsweise KOH oder gemischter MeOH liegen Carboxylgruppen auch als Lithium- oder Kaliumsalz vor.

Die im Herstellverfahren hergestellten Produkte zeichnen sich durch sehr geringe Schwenmetallgehalte aus. So liegen die Gehalte von Chrom-, Mangan-, Eisen- und Nickel-Ionen in der Summe unter 80 ppm, bevorzugt unter 60 ppm, besonders bevorzugt unter 30 ppm. Der Gehalt an Erdalkali-Ionen liegt unter 500 ppm, bevorzugt unter 200 ppm, besonders bevorzugt unter 100 ppm. Daher zeichnen sich die Produkte als wirksame Komplexiermittel für Erdalkali- und Schwermetall-Ionen aus.

In der Reaktion wird MSA in Form von Schmelze, Schuppen oder Briketts, bevorzugt Schmelze oder Schuppen, und Ammoniak flüssig, gasförmig oder im Wasser gelöst, bevorzugt flüssig oder in Wasser gelöst, eingesetzt. Wässrige Ammoniaklösungen werden mit Gehalten von >15 Gew.-%, bevorzugt >20 Gew. %, besonders bevorzugt >25 Gew. % an NH₃ eingesetzt. In der Reaktion und Aufarbeitung werden die Alkalimetallhydroxide MeOH in Substanz oder in wässriger Lösung verwendet. Wässrige Alkalimetallhydroxidlösungen werden in Konzentrationen von 10-60 Gew.-%, bevorzugt 20-55 Gew.-%, besonders bevorzugt 25-50 Gew.-% eindosiert.

In einer besonderen Ausführungsform wird MSA-Schmelze in wässrige Natronlauge bei Temperaturen von >60°C eindosiert und anschließend mit flüssigem Ammoniak oder mit konzentrierter wässriger Ammoniaklösung versetzt. Das MSA : NaOH : NH₃ : H₂O Molverhältnis beträgt dabei 2:1,5-3,5:1,5-3,5:6-20. Die Edukte werden bei Temperaturen von 90-145°C und Reaktionszeiten von 0,3-25 h umgesetzt. Aus dem Umsetzungsgemisch wird unter Zusatz von Wasser und 0,5-2,5 Mol NaOH pro 2 Mol ursprünglich eingesetztem MSA Wasser und Ammoniak bei Temperaturen von 80-135°C innerhalb von 0,5-25 h abdestilliert. Nach dem Zusatz von Wasser, mit dem Feststoffgehalte von 5-60 Gew.-% eingestellt werden, und einer Klärfiltration werden Produktlösungen erhalten, die Iminodibernsteinsäure in Ausbeuten von >73 %, Maleinsäure in Mengen von <3 %, Fumarsäure in Mengen von <10 %, Äpfelsäure in Mengen von <5 % und Asparaginsäure in Mengen von <15 % der jeweiligen theoretischen Ausbeuten beziehungsweise Mengen enthalten.

In einer weiteren besonderen Ausführungsform werden MSA-Schmelze und wässrige Natronlauge simultan und kontinuierlich im Molverhältnis von MSA : NaOH : H₂O = 2:1,5-3,5:6-20 in eine ebenso zusammengesetzte vorgelegte Maleinsäuresalzlösung oder pumpfähige Suspension bei Temperaturen von 75-125°C eindosiert. Mit Verweilzeiten von 0,1-5 h wird diese Lösung oder Suspension in einen zweiten Mischer gepumpt, in dem kontinuierlich flüssiges Ammoniak oder eine konzentrierte wässrige Ammoniaklösung hinzugefügt wird. Das Molverhältnis von MSA:Ammoniak beträgt dabei 2:1,5-3,5. Diese Lösung wird bei Temperaturen von 90-145°C und Verweilzeiten von 0,3-25 h in einem Konti-Reaktor umgesetzt. In einer Konti-Destillationskolonne wird aus dem Umsetzungsgemisch unter kontinuierlichem Zusatz von Wasser und wässriger Natronlauge, entsprechend 0,5-2,5 Mol NaOH pro 2 Mol ursprünglich eingesetztem MSA, Ammoniak und Wasser bei Temperaturen von 70-140°C und Verweilzeiten von 0,1-25 h abdestilliert. Nach dem Zusatz von Wasser werden Lösungen mit Feststoffgehalten von 5-60 Gew.-% erhalten, die gegebenenfalls einer Klärfiltration unterzogen werden. Die Produktlösungen weisen Ausbeuten von >73 % an Iminodibernsteinsäure, <3 % an Maleinsäure, <10 % an Fumarsäure, <5 % an Äpfelsäure und <15 % der jeweiligen theoretischen Ausbeuten an Asparaginsäure auf.

Im Herstellverfahren werden durch Reaktion und Aufarbeitung Produktlösungen mit einer hohen Iminodibernsteinsäure-Ausbeute und einem hohen Komplexiervermögen erhalten. Die Nebenkomponenten beeinträchtigen weder das Komplexiervermögen noch den biologischen Abbau. Die Produkte sind weitestgehend von Ammoniak befreit. Sie sind nahezu geruchsneutral, lagerstabil und weitgehend frei von störenden Erdalkali- und Schwermetall-Ionen. Nebenkomponenten treten durch Kondensation nur in sehr untergeordnetem Maße auf und werden durch die Aufarbeitung zusätzlich veringert.

Iminodibernsteinsäurealkalisalze, insbesondere die erfindungsgemäß hergestellten, sind verwendbar zur Steigerung des Weißgrades und der Helligkeit von pflanzlichen Fasern bei der Papierherstellung, beispielsweise bei der Verarbeitung von Zellstoff oder Holzstoff (Thermo Mechanical Pulp) in der Vorbehandlung der Fasern oder in der reduktiven Bleiche mit Natriumdithionit (Na₂S₂O₄), insbesonders in der Vorbehandlung der Fasen vor dem Bleichprozess oder in der reduktiven Bleiche.

### Verwendungsbeispiel

Erhöhung des Weißgrades bei der reduktiven Bleiche von Holzstoff (Thermo-Mechanical-Pulp) durch den Einsatz des Produktes aus Beispiel 6:

Der Holzstoff wurde bei einer Stoffdichte von 4 % bei 60°C mit 0,26-0,52 % Produkt (entspricht dem Feststoff) aus Beispiel 6 und unter Luftausschluss mit 1,0-1,5 % Natriumdithionit techn. (ca. 85 %ig) versetzt. Der jeweilige Mengeneinsatz bezieht sich auf den otro Faserstoff. Nach einer Bleichdauer von 1h bei 60°C (Beutelbleiche) und einem pH-Wert von 6,0 wurde der Weißgrad des TMP bestimmt. Zum Vergleich wurde ein Bleichversuch ohne Komplexiermittel durchgeführt. Folgende Ergebnisse wurden erhalten:

| Zugabe von Produkt aus Beispiel 6; | Weißgrad [%] | Weißgrad [%] |
|---|---|---|
| Feststoff [%], bez. auf otro Faserstoff | bei 1,0 % Na₂S₂O₄ | bei 1,5 % Na₂S₂O₄ |
| 0,00 | 52,3 | 53,1 |
| 0,26 | 53,6 | 54,3 |
| 0,39 | 54,0 | 54,1 |
| 0,52 | 53,8 | 53,9 |

In Gegenwart des Produktes aus Beispiel 6 ist eine Erhöhung des Weißgrades festzustellen.

## Patentansprüche

1. Verwendung von Iminodibernsteinsäurealkalisalzen in der reduktiven Bleiche mit Natriumdithionit bei der Papierherstellung.

2. Verwendung nach Anspruch 1, **dadurch gekennzeichnet, dass** die Iminodibemsteinsäurealkalisalze bei der reduktiven Bleiche von Holzstoff oder Zellstoff, eingesetzt werden.

3. Papierherstellung **gekennzeichnet durch** die Verwendung von Iminodibemsteinsäurealkalisalzen als Komplexierungsmittel für Erdalkali- und Schwermetallionen in der reduktiven Bleiche mit Natriumdithionit.

## Claims

1. Use of alkali metal salts of iminodisuccinic acid in the reductive bleach with sodium dithionite in paper production.

2. Use according to Claim 1, **characterized in that** the alkali metal salts of iminodisuccinic acid are used in the reactive bleach of mechanical pulp or chemical pulp.

3. Paper production **characterized by** the use of alkali metal salts of iminodisuccinic acid as complexing agents for alkaline earth metal and heavy metal ions in the reductive bleach with sodium dithionite.

## Revendications

1. Utilisation de sels de métal alcalin de l'acide iminodisuccinique dans le blanchiment réducteur avec du dithionite de sodium lors de la fabrication de papier.

2. Utilisation selon la revendication 1, **caractérisée en ce que** les sels de métal alcalin de l'acide iminodisuccinique sont utilisés lors du blanchiment réducteur de pâte de bois ou de pâte de cellulose.

3. Fabrication de papier **caractérisée par** l'utilisation de sels de métal alcalin de l'acide iminodisuccinique comme complexant pour les ions de métaux alcalino-terreux et de métaux lourds dans le blanchiment réducteur avec du dithionite de sodium.
